# EUROPEAN PATENT SPECIFICATION

(11) **EP 1 066 002 B1**
(45) Date of publication and mention of the grant of the patent: **09.06.2004**
(21) Application number: 99910669.3
(22) Date of filing: 24.03.1999
(51) Int. Cl.: A61F 6/00

(54) **DEVICE AND METHOD FOR THE FACILITATED INSERTION OF THE MALE MEMBER INTO A CONDOM**
VORRICHTUNG UND VERFAHREN ZUR VEREINFACHTEN EINFÜHRUNG EINES MÄNNLICHEN GLIEDES IN EIN KONDOM
DISPOSITIF ET PROCEDE POUR FACILITER L'INSERTION DU PENIS DANS UN PRESERVATIF

(30) Priority: 27.03.1998 IT RM980199
(43) Date of publication of application: 10.01.2001
(73) Proprietor: Ruggero, Vincenzo, 80078 Pozzuoli (IT); Di Camillo, Lorenzo, 03040 San Vittore del Lazio (IT)
(72) Inventor: DI CAMILLO, Lorenzo, I-03040 San Vittore del Lazio (IT)
(74) Representative: Leone, Mario
(86) International application number: PCT/IT1999/000070
(87) International publication number: WO 1999/049821

(56) References cited:
- DE-A- 4 241 441
- GB-A- 2 290 972
- NL-A- 9 100 675
- US-A- 4 984 582
- US-A- 5 471 998

## Description

The present invention relates to a device and a method for the facilitated insertion of the male member into a condom.

At present the insertion of a condom onto the male member is carried out by leaning the brim of the same condom over the erected male member and subsequently unrolling the condom along the whole length of the organ.

A first drawback connected to said very common insertion method is the possibility, not at all remote, of breaking the condom with one's nails: very often, in fact, a correct insertion cannot be carried out by simply unrolling the condom, with the consequence of a prolonged handling of the same condom and higher probability of damaging the latter with one's nails.

A second drawback is the impossibility to know for certain whether the condom presents perforations that would make its use unadvisable. Very often, in fact, breaking of the condom during the sexual intercourse is not so much due to prolonged use or possible problems which may increase friction, as to pre-existing defects.

A further drawback is given by the fact that it is difficult to insert the condom in the absence of a complete erection.

Some kind of devices for the insertion of the male member into a condom are known from NL-A-9 100 675, DE-A-4 241 441 and GB-A-2 290 972.

Such known devices are all based on the same principle: the creation of a depression inside the device that cause the condom to inflate and allows the insertion of the male member into the inflated condom.

Nevertheless, such devices have an important drawback. When the depression is created also the receptacle of the condom is inflated, so that the resulting dressing of the condom is not correct and it can not operate properly.

The present invention overcomes such prior art drawbacks as it provides a device for the facilitated insertion of the male member into a condom, comprising:
- a hollow element for containing the condom, provided with an access aperture;
- means for fastening a brim of the condom to the access aperture of the hollow element, in order to form an air chamber between external walls of the condom and internal walls of the hollow element; and
- means, associated with said hollow element, for creating a depression inside said air chamber forcing adhesion of the condom to the internal walls of the hollow element and allowing the subsequent facilitated insertion of the male member,
the hollow element being provided with mobile walls, said depression resulting from the increased volume of the hollow element,
the device being characterised in that it further comprises
- a bearing element located inside the hollow element and having a bearing plane for bearing a base of the condom.
A method for the facilitated insertion of the male member into a condom is further provided, comprising the steps of:
- inserting the condom into a hollow element so as to form an air chamber between external walls of the condom and internal walls of the hollow element;
- creating a depression in said air chamber, forcing adhesion of the condom to the internal walls of the hollow element, said depression being obtained by increasing the volume of the hollow element;
- inserting the male member inside the internal
- area of the condom; and
- removing the condom from the hollow element, in order for said condom to completely adhere to the male member,
characterised in that it further comprises the step of
- providing a bearing element having a bearing plane for bearing a base of the condom before said step of creating a depression.

Advantageous features of the present invention are claimed in the dependent claims thereof.

In this way, the above-mentioned problems are solved as not only the manipulation of the condom during the insertion onto the male member is almost completely reduced, but moreover the presence of possible perforations in the condom is immediately detected. In fact, the depression which is created would in such case cause the immediate breaking of the condom.

A further advantage of the present invention is given by the fact that it is moreover possible to insert the condom even in the absence of a full erection.

A still further advantage is given by the fact that the device and the method according to the present invention, given the easiness with which they can be used, are particularly helpful to invalid, handicapped and disabled people in general.

The present invention will be hereinafter disclosed by preferred embodiments thereof, shown as non-limiting examples.

Reference will be made to the annexed drawings wherein:
figure 1 is a partial perspective view of a first embodiment of the device according to the present invention;
figure 2 is a section view of the embodiment of figure 1, with an inserted condom;
figure 3 is a partial perspective view of a second embodiment of the device according to the present invention, with an inserted condom and in a state of minimum extension;
figure 4 is a section view of the embodiment of figure 3, in a state of maximum extension;
figure 5 shows a partial perspective and exploded view of a third embodiment of the device; and
figures 6 to 8 show how to use the device in the embodiment of figure 5.

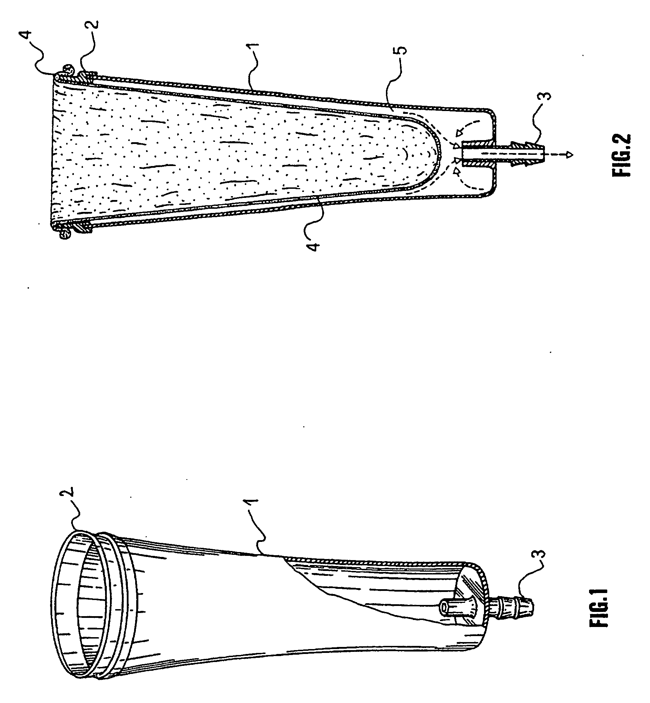

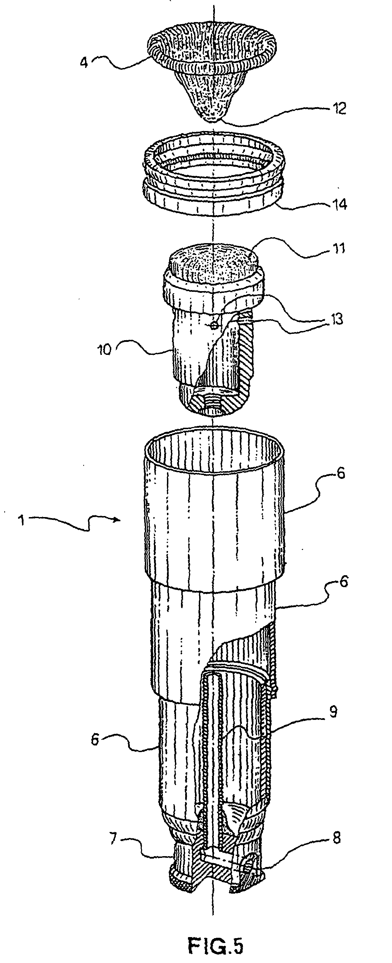

Figure 1 shows a hollow element 1 for containing a condom provided with an access aperture, located on top in the figure, which shows means for fastening the condom to the hollow chamber such as a fastening ring 2 to which the brim of the condom has to be fastened. In this embodiment the ring 2 is integrally formed with the hollow element 1, being made up by the peripheral rim of the access aperture of the hollow element. For a different kind of ring, figures 5 to 8 are addressed. A suction duct 3, whose aim will be clarified by the detailed explanation of subsequent figure 2, is also shown in figure 1, which duct is in contact with the bottom of the hollow element 1.

Figure 2 shows a section view of the hollow element 1 of figure 1 with an inserted condom 4. The brim of the condom 4 is fastened to the fastening ring 2. When the condom 4 is inserted, an air chamber 5 is formed between the external walls of the condom 4 and the internal walls of the hollow element 1. The presence of the suction duct 3 is intended for creating a depression in the air chamber 5, as schematically illustrated by the dotted arrows in the figure; such depression will force the condom 4 to adhere to the internal walls of the hollow element 1 allowing the subsequent facilitated insertion of the male member.

Depression inside the hollow element 1 can be obtained according to various modes. A first mode involves providing the suction duct 3 with a non-return air valve, not shown in the figure. A second mode involves the suction duct being made up of flexible walls, so that it can be closed by throttling. It is to be intended that in this case the suction duct can be advantageously lengthened in order to facilitate the user in the air-sucking operation. It is to be understood that other means for creating depression inside the air chamber could be easily detected by the skilled person.

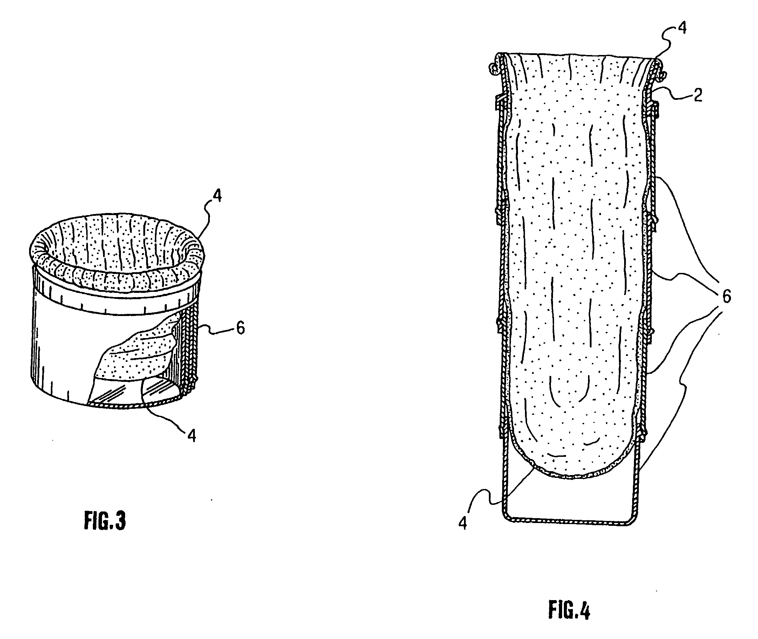

Subsequent figure 3 shows an alternative embodiment of the present invention, wherein the hollow element 1 is such as to involve a plurality of mobile walls 6, articulated in a telescopic relation and shown in a condition of minimum extension. This kind of embodiment allows a considerable reduction of the space taken by the device. In this case, the depression will be obtained by increasing the volume of the hollow element 1, starting from a condition of minimum extension until reaching a condition of maximum extension, shown in subsequent figure 4, wherein the adherence of the external walls of the condom 4 to the internal side of the mobile walls 6 of the hollow element 1 caused by the depression is easily seen. Embodiments with mobile walls, not necessarily in a telescopic relation, could also be provided.

A simple way to carry out the fastening operation in all embodiments illustrated up to this point will be to widen the brim of the condom by hand until it will be wider than the diameter of the fastening ring, releasing thereafter said brim so as to allow fastening of the brim of the condom to the external area adjacent to the fastening ring.

After the insertion of the male member, the brim of the condom will be easily removed from the fastening element, so as to eliminate the adherence of the condom to the hollow element and allow it to adhere completely to the male member.

In order to further facilitate the adherence of the condom to the male member it is moreover possible to provide means apt to re-establish, after the insertion, the internal pressure which existed previously to the above described depression. If, for example, the suction duct has been closed by throttling, it will be sufficient to reopen it. In this way the problems that may possibly arise during the removal of the device in case of male members of small size are solved.

Besides the above-mentioned advantages connected with checking the integrity of the condom and avoiding breaking the condom unintentionally with one's nails, further advantages are represented by a higher hygiene and a considerably facilitated insertion, in particular for users endowed with members of large size.

An alternative embodiment of the present invention involves moreover an additional protective sheath or equivalent means, disposed along the internal walls of the hollow element 1, which can be advantageously removed after the insertion of the condom. In this way the contact between the condom and the internal walls of the hollow element is avoided, in order to guarantee even higher hygienic conditions.

A further embodiment of the present invention provides for the facilitated insertion device to comprise fastening rings of the disposable type, with a pre-inserted condom. These rings are intended to be normally independent from the rest of the hollow body, and connectable therewith only during use. Therefore, a new fastening ring with its respective condom will have to be inserted for each subsequent use.

Reference will now be made to figures 5 to 8, showing a further embodiment of the device according to the invention. This embodiment makes use of a fastening ring of the disposable type together with means apt to re-establish, after the insertion, the internal pressure which existed previously to the depression.

Figure 5 shows a partial perspective and exploded view of the device, having mobile walls 6, articulated in a telescopic relationship (represented in figure in a condition of maximum extension), together with a base element 7, having a diameter which is inferior to the minor of the diameters of the mobile walls 6. The base element comprises a hole 8 allowing air passage from the outside to the inside of the device and is internally connected to a screw 9, which is therefore placed internally to the hollow element 1 . Also the screw 9 is hollow, in order to consent the air passage from the outside to the inside of the device and vice versa. The function of the screw 9 is that of allowing the screwing onto it of a cylindrical element 10, the function thereof being that of providing a bearing plane 11 for the base 12 of the condom 4, related to the receptacle region thereof.

In fact, it has been discovered by the applicants that the presence of such a bearing element is advantageous, as thus a slight preliminary squashing of the receptacle prior to the depression phase can be obtained, and the possible risk of its inflating is in this way significantly reduced.

The bearing element 10 is screwed onto the screw 9, and it comprises holes 13 allowing the air passage between the mouth of the hollow screw 9 and the inside of the hollow element 1. The elevation of the bearing element 10 inside the hollow element 1 is adjustable, as it simply suffices to screw it more, or less, onto the screw 9. Thus, a convenient bearing base 11 can be realized for condoms of any length.

In the embodiment of figure 5, the condom 4 is to be intended as inserted inside of a ring 14, disjointed from the hollow element 1 and apt to be inserted, for instance by fixing, onto the upper brim thereof.

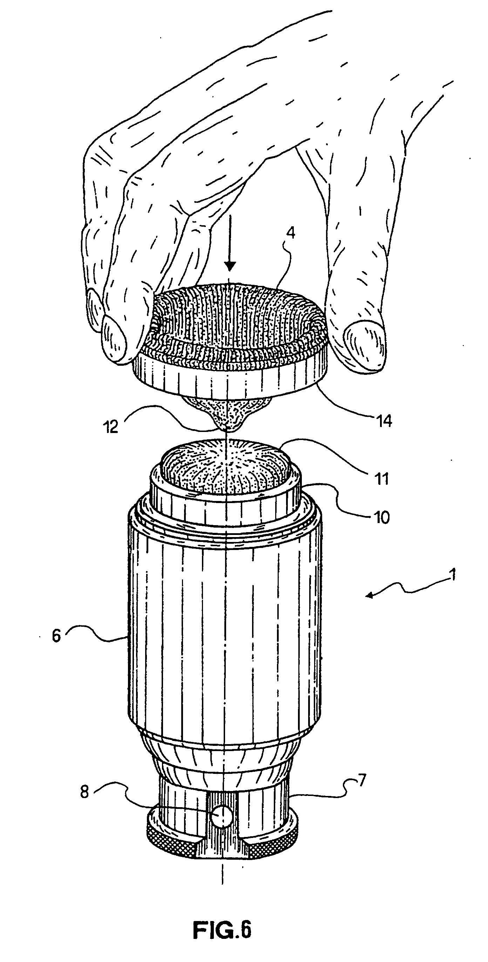

The next figures 6 to 8 show how to use the device in the embodiment of figure 5.

Figure 6 shows how the initial insertion by fixing of the ring 14 onto the element 1 is carried out, therefore ensuring that the base/receptacle 12 of the condom 4 comes into contact with the bearing plane 11 of the element 10. The mobile walls 6 of the hollow element 1 are here shown in a condition of minimal extension.

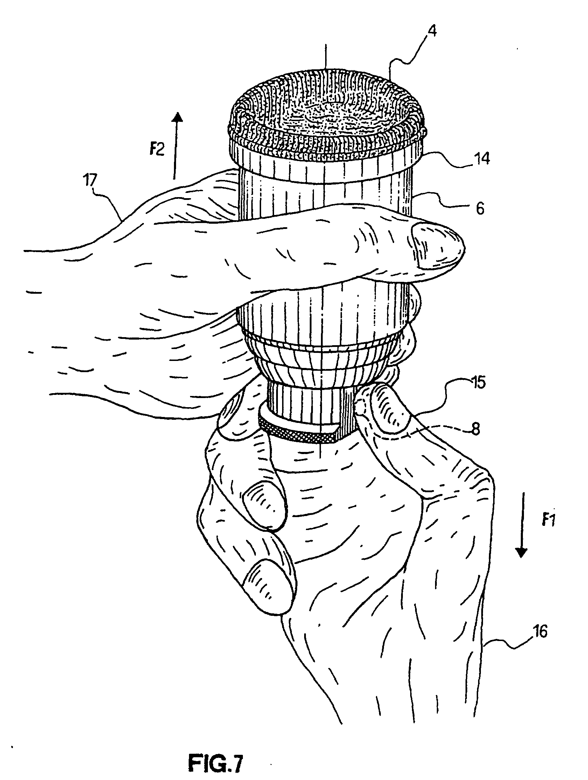

Figure 7 shows the operation for creating a depression inside the inner air chamber. In particular the position of the user's thumb on the hole 8 has to be noted, to the end of succeeding in the depression operation at issue. The volume increase in order to cause the depression can be obtained either by pulling the hand 16 in the direction of the arrow F1, or pulling the hand 17 in the direction of the arrow F2.

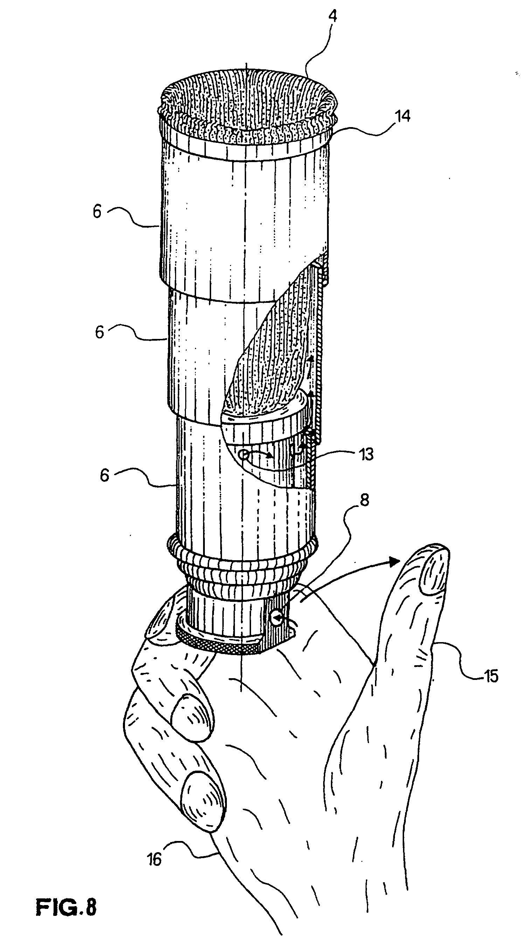

Figure 8 firstly shows how, as a consequence of the operation described above, the outer walls of the condom have adhered to the inner walls of the hollow element. Then the condom shall be inserted onto the male member. Once this insertion (not shown in figure) has been carried out, the thumb 15 is released in order to re-establish the internal pressure which existed previously to the depression, so as to allow a first tightening of the condom onto the male member, and then the brim of the condom shall be removed off the ring 14, to ensure the complete adhering thereof.

It is to be intended that various and different are the possible modifications to the embodiments illustrated up to this point, all of them, however, falling within the protective scope of the present invention. For example, embodiments can be provided wherein the fastening ring 2 is not provided as an additional element, but is integrally formed with the hollow element 1, being in this case made up by the peripheral rim of the access aperture of the hollow element.

## Claims

1. A device for the facilitated insertion of the male member into a condom (4), comprising:
- a hollow element (1) for containing the condom (4), provided with an access aperture;
- means (2; 14) for fastening a brim of the condom (4) to the access aperture of the hollow element (1), in order to form an air chamber (5) between external walls of the condom (4) and internal walls of the hollow element (1); and
- means, associated with said hollow element (1), for creating a depression inside said air chamber (5) forcing adhesion of the condom (4) to the internal walls of the hollow element (1) and allowing the subsequent facilitated insertion of the male member,
the hollow element (1) being provided with mobile walls (6), said depression resulting from the increased volume of the hollow element (1),
the device being **characterised in that** it further comprises
- a bearing element (10) located inside the hollow element (1) and having a bearing plane (11) for bearing a base (12) of the condom (4).

2. The device according to claim 1, **characterized in that** said means for creating a depression comprises a suction duct (3) provided with a non-return valve.

3. The device according to claim 1, **characterized in that** said means for creating a depression comprises a suction duct with flexible walls, the duct being apt to be closed by throttling.

4. The device according to any of the preceding claims, **characterized in that** said mobile walls (6) are articulated in a telescopic relation therebetween.

5. The device according to any of the preceding claims, **characterized in that** the elevation of the bearing element (10) inside the hollow element (1) is adjustable.

6. The device according to any of the preceding claims, **characterized in that** it comprises means for avoiding contact between the external walls of the condom (4) and the internal walls of the hollow element (1).

7. The device according to claim 6, **characterized in that** said means for avoiding contact are removable.

8. The device according to any of the preceding claims, **characterized in that** said means (2) for fastening a brim of the condom (4) to the access aperture of the hollow element (1) are integrally formed therewith.

9. The device according to any of the preceding claims, **characterized in that** it comprises means for re-establishing, after said insertion, the internal pressure existing before the depression.

10. A method for the facilitated insertion of the male member into a condom (4), comprising the steps of:
- inserting the condom (4) into a hollow element (1) so as to form an air chamber (5) between external walls of the condom (4) and internal walls of the hollow element (1);
- creating a depression in said air chamber (5), forcing adhesion of the condom to the internal walls of the hollow element (1), said depression being obtained by increasing the volume of the hollow element (1);
- inserting the male member inside the internal area of the condom (4); and
- removing the condom (4) from the hollow element (1), in order for said condom (4) to completely adhere to the male member,
**characterised in that** it further comprises the step of
- providing a bearing element (10) having a bearing plane (11) for bearing a base (12) of the condom (4) before said step of creating a depression.

11. The method according to claim 10, **characterized in that** said depression is obtained by suction of the air contained inside said hollow element (1).

12. The method according to claim 10 or 11, **characterized in that** it furthermore comprises a step for re-establishing, after the removal of the condom (4) from the hollow element (1), the internal pressure existing before the depression.

## Patentansprüche

1. Vorrichtung zum vereinfachten Einführen des männlichen Gliedes in ein Kondom (4), die umfasst:
ein hohles Element (1) zum Aufnehmen des Kondoms (4), das mit einer Zugangsöffnung versehen ist;
eine Einrichtung (2; 14) zum Befestigen eines Randes des Kondoms (4) an der Zugangsöffnung des hohlen Elementes (1), um eine Luftkammer (5) zwischen Außenwänden des Kondoms (4) und Innenwänden des hohlen Elements (1) auszubilden; und
eine mit dem hohlen Element (1) verbundene Einrichtung zum Erzeugen eines U n-terdrucks im Inneren der Luftkammer (5), der Haftung des Kondoms (4) an den Innenwänden des hohlen Elementes (1) erzwingt und das anschließende vereinfachte Einführen des männlichen Gliedes ermöglicht,
wobei das hohle Element (1) mit beweglichen Wänden (6) versehen ist und der Unterdruck durch das vergrößerte Volumen des hohlen Elementes (1) verursacht wird,
wobei die Vorrichtung **dadurch gekennzeichnet ist, dass** sie des Weiteren umfasst:
ein Aufliegeelement (10), das sich im Inneren des hohlen Elementes (1) befindet und eine Aufliegeebene (11) zum Aufliegen eines unteren Endes (12) des Kondoms (4) hat.

2. Vorrichtung nach Anspruch 1, **dadurch gekennzeichnet, dass** die Einrichtung zum Erzeugen eines Unterdrucks eine Absaugleitung (3) umfasst, die mit einem Rückschlagventil versehen ist.

3. Vorrichtung nach Anspruch 1, **dadurch gekennzeichnet, dass** die Einrichtung zum Erzeugen eines Unterdrucks eine Ansaugleitung mit flexiblen Wänden umfasst,
wobei die Leitung durch Drosselung geschlossen werden kann.

4. Vorrichtung nach einem der vorangehenden Ansprüche, **dadurch gekennzeichnet, dass** die beweglichen Wände (6) miteinander teleskopartig gelenkig verbunden sind.

5. Vorrichtung nach einem der vorangehenden Ansprüche, **dadurch gekennzeichnet, dass** die Höhe des Aufliegelementes (10) im Inneren des hohlen Elementes (1) verstellt werden kann.

6. Vorrichtung nach einem der vorangehenden Ansprüche, **dadurch gekennzeichnet, dass** sie eine Einrichtung zum Verhindern von Kontakt zwischen den Außenwänden des Kondoms (4) und den Innenwänden des hohlen Elementes (1) umfasst.

7. Vorrichtung nach Anspruch 6, **dadurch gekennzeichnet, dass** die Einrichtung zum Verhindern von Kontakt entfernt werden kann.

8. Vorrichtung nach einem der vorangehenden Ansprüche, **dadurch gekennzeichnet, dass** die Einrichtung (2) zum Befestigen eines Randes des Kondoms (4) an der Zugangsöffnung des hohlen Elementes (1) integral damit ausgebildet ist.

9. Vorrichtung nach einem der vorangehenden Ansprüche, **dadurch gekennzeichnet, dass** sie eine Einrichtung umfasst, mit der der innere Druck, der vor dem U n-terdruck herrschte, nach dem Einführen wiederhergestellt wird.

10. Verfahren zum vereinfachten Einführen des männlichen Gliedes in ein Kondom (4), das die folgenden Schritte umfasst:
Einführen des Kondoms (4) in ein hohles Element (1), um so eine Luftkammer (5) zwischen Außenwänden des Kondoms (4) und Innenwänden des hohlen Elements (1) auszubilden;
Erzeugen eines Unterdrucks in der Luftkammer (5), der Haftung des Kondoms an den Innenwänden des hohlen Elementes (1) erzwingt, wobei der Unterdruck hergestellt wird, indem das Volumen des hohlen Elementes (1) vergrößert wird;
Einführen des männlichen Gliedes in den Innenbereich des Kondoms (4); und
Entfernen des Kondoms (4) aus dem hohlen Element (1), damit das Kondom (4) vollständig an dem männlichen Glied haftet,
**dadurch gekennzeichnet, dass** es des Weiteren den Schritt des Bereitstellens eines Aufliegelementes (10) mit einer Aufgegebene (11) umfasst, auf der ein unteres Ende (12) des Kondoms (4) vor dem Schritt des Erzeugens eines Unterdrucks aufliegt.

11. Verfahren nach Anspruch 10, **dadurch gekennzeichnet, dass** der Unterdruck durch Absaugen der im Inneren des hohlen Elementes (1) enthaltenen Luft erreicht wird.

12. Verfahren nach Anspruch 10 oder 11, **dadurch gekennzeichnet, dass** es des Weiteren einen Schritt zum Wiederherstellen des vor dem Unterdruck herrschenden Innendrucks nach dem Entfernen des Kondoms (4) aus dem hohlen Element (1) umfasst.

## Revendications

1. Dispositif pour l'insertion facilitée de l'organe mâle dans un préservatif (4), comportant :
- un élément creux (1) destiné à contenir le préservatif (4), muni d'une ouverture d'accès,
- des moyens (2 ; 14) pour fixer un bord du préservatif (4) sur l'ouverture d'accès de l'élément creux (1), afin de former une chambre à air (5) entre des parois extérieures du préservatif (4) et des parois intérieures de l'élément creux (11), et
- des moyens, associés audit élément creux (1), pour créer une dépression à l'intérieur de ladite chambre d'air (15), imposant la mise en adhérence du préservatif (4) sur les parois intérieures de l'élément creux (1), et permettant l'insertion facilitée consécutive de l'organe mâle,
l'élément creux (1) étant muni de parois mobiles (6), ladite dépression étant le résultat du volume accrû de l'élément creux (1),
le dispositif étant **caractérisé en ce qu'**il comporte de plus
- un élément de support (10) positionné à l'intérieur de l'élément creux (1), et ayant un plan de support (11) destiné à supporter une base (12) du préservatif (4).

2. Dispositif selon la revendication 1, **caractérisé en ce que** lesdits moyens pour créer une dépression comportent un conduit d'aspiration (3) muni d'un clapet antiretour.

3. Dispositif selon la revendication 1, **caractérisé en ce que** lesdits moyens pour créer une dépression comportent un conduit d'aspiration muni de parois souples, le conduit étant apte à être fermé par étranglement.

4. Dispositif selon l'une quelconque des revendications précédentes, **caractérisé en ce que** lesdites parois mobiles (6) sont articulées selon une relation télescopique entre celles-ci.

5. Dispositif selon l'une quelconque des revendications précédentes, **caractérisé en ce que** la hauteur de l'élément de support (10) à l'intérieur de l'élément creux (1) est ajustable.

6. Dispositif selon l'une quelconque des revendications précédentes, **caractérisé en ce qu'**il comporte des moyens pour éviter un contact entre les parois extérieures du préservatif (4) et les parois intérieures de l'élément creux (1).

7. Dispositif selon la revendication 6, **caractérisé en ce que** lesdits moyens pour éviter un contact sont amovibles.

8. Dispositif selon l'une quelconque des revendications précédentes, **caractérisé en ce que** lesdits moyens (2) pour fixer un bord du préservatif (4) sur l'ouverture d'accès de l'élément creux (1) sont formés en un seul bloc avec celui-ci.

9. Dispositif selon l'une quelconque des revendications précédentes, **caractérisé en ce qu'**il comporte des moyens pour établir à nouveau, après insertion, la pression interne existant avant la dépression.

10. Procédé pour l'insertion facilitée de l'organe mâle dans un préservatif (4), comportant les étapes consistant à :
- insérer le préservatif (4) dans un élément creux (1), de manière à former une chambre à air (5) entre des parois extérieures du préservatif (4) et des parois intérieures de l'élément creux (1),
- créer une dépression dans ladite chambre à air (5), imposant la mise en adhérence du préservatif sur les parois intérieures de l'élément creux (1), ladite dépression étant obtenue en augmentant le volume de l'élément creux (1),
- insérer l'organe mâle à l'intérieur de la zone intérieure du préservatif (4), et
- enlever le préservatif (4) à partir de l'élément creux (1), pour que ledit préservatif (4) vienne entièrement en adhérence sur l'organe mâle,
**caractérisé en ce qu'**il comporte de plus l'étape consistant à
- fournir un élément de support (10) ayant un plan de support (11) destiné à supporter une base (12) du préservatif (4) avant ladite étape consistant à créer une dépression.

11. Procédé selon la revendication 10, **caractérisé en ce que** ladite dépression est obtenue par aspiration de l'air contenu à l'intérieur dudit élément creux.

12. Procédé selon la revendication 10 ou 11, **caractérisé en ce qu'**il comporte de plus une étape consistant à établir à nouveau, après enlèvement du préservatif (4) à partir de l'élément creux (1), la pression interne existant avant la dépression.
